## Europäisches Patentamt
## European Patent Office
## Office européen des brevets

(11) Publication number: **0 021 800**

**A2**

# EUROPEAN PATENT APPLICATION

(21) Application number: **80302075.9**

(22) Date of filing: **19.06.80**

(51) Int. Cl.³: **A 61 B 5/02**
**G 04 G 1/00**

(30) Priority: **21.06.79 GB 7921600**

(43) Date of publication of application:
**07.01.81 Bulletin 81/1**

(84) Designated Contracting States:
**AT BE CH DE FR IT LI LU NL SE**

(71) Applicant: **PULSE TIME UK LIMITED**
**The Centre High Street**
**Weybridge Surrey KT13 8BL(GB)**

(72) Inventor: **Carruthers, Malcolm E.**
**12 Milford House 7 Queen Anne Street**
**London W1M 9FD(GB)**

(74) Representative: **Thomas, Christopher Hugo et al,**
**D Young & Co 10 Staple Inn**
**London WC1V 7RD(GB)**

(54) **Cardiovascular monitors.**

(57) A cardiovascular monitor comprises a watch casing, a timing circuit (3) in the watch casing for supplying timing signals, a transducer (2) associated with the watch casing for sensing arterial blood pulses and supplying an electric signal in dependence thereon, electrodes (7) associated with the watch casing for sensing electrocardiographic R-waves and supplying an electric signal in dependence thereon, a circuit (5) for deriving from the transducer signal and a timing signal an electric signal representing heartbeat rate, a circuit (4) for deriving from the electrode signal, the transducer signal and a timing signal an electric signal representing mean blood pressure, and a display (6) on the watch casing for displaying the time of day in dependence on a timing signal, heartbeat rate in dependence on the heartbeat rate signal, and mean blood pressure in dependence on the blood pressure signal. Alternatively, only the time of day and the heartbeat rate, or only the mean blood pressure may be displayed.

FIG. 2

1

## CARDIOVASCULAR MONITORS

This invention relates to cardiovascular monitors.

There has previously been proposed in UK patent no. 1 312 107 a heartbeat rate monitor which includes a watch casing containing a timing circuit, a transducer for the detection of heartbeats, a comparator circuit for deriving from signals supplied by the timing circuit and the transducer a further signal representing the heartbeat rate per unit time, and display means for displaying the time of day and the heartbeat rate. UK patent no. 1 542 850 discloses a transducer for use in such a heartbeat rate monitor, and UK patent no. 1 555 062 discloses a solar-powered heartbeat rate monitor which may also form a watch.

According to the present invention there is provided a cardiovascular monitor comprising:

a casing;

a timing circuit disposed in said casing for supplying a timing signal;

a transducer associated with said casing for sensing arterial blood pulses at said transducer and supplying an electric signal in dependence thereon;

electrode means associated with said casing for sensing electrocardiographic R-waves at said electrode means and supplying an electric signal in dependence thereon;

circuit means for deriving from said electrode signal, said transducer signal and said timing signal an electric signal representing mean blood pressure; and

display means mounted on said casing for displaying mean blood pressure in dependence on said blood pressure signal.

If desired, embodiments of the invention can be generally in the form of a watch and provide readings of the time of day and the heartbeat rate, in addition to the blood pressure.

According to the present invention, therefore, there is also provided a cardiovascular monitor comprising:

a watch casing;

a timing circuit disposed in said watch casing for supplying timing signals;

a transducer associated with said watch casing for sensing arterial blood pulses at said transducer and supplying an electric signal in dependence thereon;

2

electrode means associated with said watch casing for sensing electrocardiographic R-waves at said electrode means and supplying an electric signal in dependence thereon;

circuit means for deriving from said transducer signal and a said timing signal an electric signal representing heartbeat rate;

circuit means for deriving from said electrode signal, said transducer signal and a said timing signal an electric signal representing mean blood pressure; and

display means mounted on said watch casing for displaying the time of day in dependence on a said timing signal, heartbeat rate in dependence on said heartbeat rate signal, and mean blood pressure in dependence on said blood pressure signal.

Embodiments of the invention derive said blood pressure signal by determining the interval between the R-wave which, being an electric signal is transmitted substantially instantaneously from the heart to all parts of the body, and the foot or other constant point of the corresponding arterial pulse, normally the foot of the corresponding radial pulse. This interval is the transit time for the pressure pulse to travel from the heart to the transducer. This transit time is inversely proportional to the mean blood pressure and so enables the blood pressure signal to be derived.

It will be realised that said electrode signal is periodic in just the same way as said transducer signal, and in alternative embodiments where blood pressure measurement is not required it can be used in place of said transducer signal for deriving said heartbeat rate signal, so making said transducer unnecessary.

According to the present invention, therefore, there is also provided a cardiovascular monitor comprising:

a watch casing;

a timing circuit disposed in said watch casing for supplying timing signals;

electrode means associated with said watch casing for sensing electrocardiographic R-waves at said electrode means and supplying an electric signal in dependence thereon;

circuit means for deriving from said electrode signal and a said timing signal an electric signal representing heartbeat rate; and

display means mounted on said watch casing for displaying the time of day in dependence on a said timing signal, and heartbeat rate in dependence on said heartbeat rate signal.

Where the above-mentioned said transducer is provided, the possibility exists of using the transducer for solar or other ambient light recharging of a battery in the monitor. In this case said transducer comprises a light source for transilluminating skin tissue and semiconductor detector means disposed to receive light from said light source reflected from skin tissue and capable of detecting variations in the level of said light reflected from said skin tissue so as to produce said transducer signal which is responsive to changes in arterial blood flow in said skin tissue, said detector means also being capable, on exposure to ambient light, of supplying a recharging current for a power source; and said monitor further comrises a rechargeable electric power source; means to supply said current from said detector means to said power source to recharge said power source; and means to energise said timing circuit, light source, circuit means and display means from said power source.

The invention will now be described by way of example with reference to the accompanying drawings, in which:

Figure 1 shows the waveforms of an electrocardiographic pulse and an arterial pulse; and

Figure 2 shows in block form an embodiment of cardiovascular monitor according to the invention.

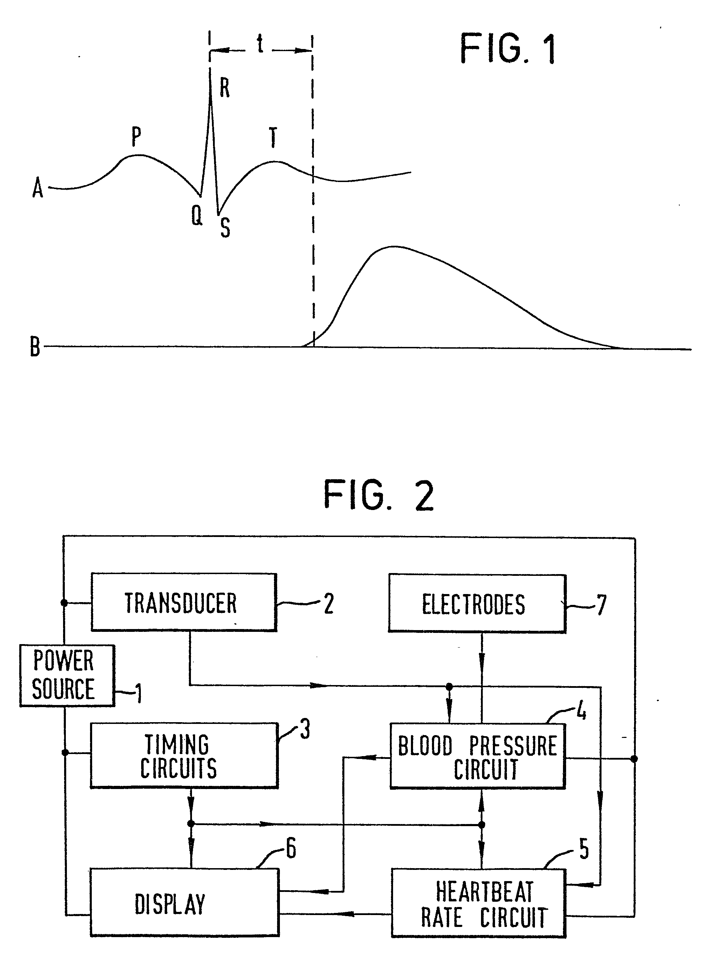

The basic theory of the method used for blood pressure measurement will first be described. Reference is made to Figure 1 in which waveform A is an electrocardiographic trace for a single heartbeat, the ordinates being voltage, and waveform B is a trace of the pressure variation at a point in an artery remote from the heart, say at the wrist or fingertips, and corresponding to the same single heartbeat. The ordinates for waveform B are pressure, and for waveforms A and B the abscissae are time to the same scale. The maxima and minima of the waveform A are referenced P, Q, R, S, and T using the standard notation, and the part of the waveform which is of particular interest in the present context is the maximum R referred to herein as is usual as the R-wave.

Because the waveform A represents the electrocardiographic trace, that is to say an electric signal, it appears at all points of the body at substantially the same time as it is generated and applied to the heart muscle. Of course the voltage decreases with distance from the heart, but it has been found that a usable, detectable voltage, amounting to approximately one-half millivolt for the R-wave occurs between the extremities of the arms.

The foot of the pulse in the waveform B lags behind the R-wave by a time which is dependent on the pulse wave velocity in the arteries and the distance from the heart. It has been shown that:

$$C^2 = \frac{V}{\rho} \cdot \frac{\triangle P}{\triangle V}$$

where:      C      is the pulse wave velocity

                V      is the initial volume

                $\rho$      is the density of blood

                $\triangle P$    is the change in pressure

                $\triangle V$    is the change in volume.

Since the relationship between changes in the pressure and the volume is substantially linear over the physiological range, it follows that changes in the pulse wave velocity are dependent on changes in the arterial pressure. Moreover, the transit time $\underline{t}$ in Figure 1, that is to say the interval between the R-wave and the foot of the pulse is inversely proportional to the arterial pressure. In other words, the transit time $\underline{t}$ is inversely proportional to the mean blood pressure, and measurement of the transit time provides a measure of the mean blood pressure.

An embodiment of cardiovascular monitor according to the invention will now be described with reference to Figure 2, which shows a block diagram of the monitor. The monitor is preferably formed as a wrist or pocket watch with the various integers to be described mounted in or on the casing of the watch and/or on an associated watch strap as appropriate. The monitor comprises a power source 1 in the form of a battery which supplies power to a transducer 2, timing circuits 3, a blood pressure circuit 4, a heartbeat rate circuit 5 and a display 6. Finally, the monitor includes electrodes 7. All the circuits are integrated circuits, and may be formed as a single or at least a small number of chips.

The transducer 2 is for detecting arterial pulses and supplying an electric signal in dependence thereon to the blood pressure circuit and the heartbeat rate circuit. The transducer preferably comprises a light emitting diode for emitting light of wavelengths in the range 500 to 650 nm, and preferably in the range 600 to 650 nm into skin tissue, and a light-sensitive means such as photodiodes or photodetectors arranged, for example, in an array encircling the light emitting diode, to receive light reflected from the skin tissue. The transducer 2 may be as described in patent no. 1 542 850 or patent no. 1 555 062, and in the latter case the photodiodes are arranged not only to cooperate with the light emitting diode to detect light reflected from the skin tissue, but also in an alternative mode of operation to be exposed to ambient light to generate a current for recharging the power source, where the power source 1 is a rechargeable battery. Means may be required to step-up the voltage supplied by the photodiodes when the current is to be used for recharging the power source 1, and a blocking diode may be required to prevent the power source 1 from discharging through the photodiodes when in darkness or not otherwise in the charging mode.

The transducer 2 can be mounted on the rear of the watch casing so as always to be in contact with the skin tissue, in which case it is preferably surrounded by a seal to exclude ambient light. Alternatively, and particularly where the recharging facility is included, it can be mounted on an exposed surface of the watch casing or strap where a finger of the user can be placed over it when a reading is to be taken. In this case, the shape and configuration of the transducer 2 is arranged to be such that the finger effects the necessary exclusion of ambient light.

As an alternative, the transducer 2 may detect arterial pulses and supply an electric signal in dependence thereon using a Doppler ultrasonic technique in which the change in the frequency of the sound made by the blood as it approaches and passes the transducer 2 is detected.

The electrodes 7 conveniently comprise the metallic watch casing itself and a second metallic electrode mounted on an exposed surface of the watch casing and insulated therefrom. The housing of the transducer could itself form the required electrode when the monitor is being used to indicate blood pressure. This provides, in effect, a capacitor across which a voltage is developed on the occurrence of the R-wave when a finger of the opposite hand is placed on the second electrode.

The timing circuits 3 comprise a crystal-controlled oscillator with associated dividing circuits to provide signals supplied to the display 6 for the purpose of indicating the time at least in hours, minutes and seconds. The timing circuits 3 are to this extent therefore of the same form as in known

6

electronic watches, but are not limited thereto.

The display 6 is a numeric display and may be formed of light emitting diodes or liquid crystal cells in figure-of-eight configuration. Liquid crystal cells are preferred, because of the lower power consumption. Provision is made for switching the display 6 between the display of the time, blood pressure and heartbeat rate as required. Alternatively, separate displays may be provided. An indicator which simply flashes in synchronism with the heartbeats may also be provided.

The blood pressure circuit 4 receives an electric signal from the transducer 2 in dependence on the detected arterial blood pulses, an electric signal from the electrode 7 dependent on the periodic R-waves, and a signal from the timing circuits 3. From these input signals the blood pressure circuit derives the transit time $t$, and hence an output electric signal directly proportional to the mean blood pressure for supply to the display 6.

In a normal case, where the mean blood pressure is 100, expressed in mm of mercury, the transit time $t$ measured at the wrist will be approximately 200 milliseconds. One method of deriving the output electric signal representing the mean blood pressure is based on the fact that 200 milliseconds corresponds to a notional frequency of 300 per minute, and division of this figure by 3 gives a figure of 100, which corresponds to the mean blood pressure. As the mean blood pressure increases, the transit time $t$ decreases, so the notional frequency increases and the derived mean blood pressure figure increases as required. The required effect is also achieved when the mean blood pressure decreases.

Of course these mean blood pressure measurements although precisely related to the actual mean blood pressure, do not show the actual mean blood pressure. If required, a specific cardiovascular monitor can be calibrated for a particular subject by measurement of the actual mean blood pressure of the subject and adjustment of the monitor to show this reading. In many cases, however, it is changes of blood pressure, rather than actual blood pressure which is of particular interest, and in such cases calibration is unnecessary.

The heartbeat rate circuit 5 receives only the electric signal from the transducer 2 and an electric signal from the timing circuits 3, and in dependence thereon supplies an output signal dependent on the heartbeat rate for supply to the display 6. The output signal will preferably represent a form of moving average of the frequency in pulses per minute of the electric signal from the transducer 2. The moving average may be taken over predetermined time intervals or in respect of predetermined numbers of successive pulses, and some compromise is necessary to resolve the conflict between the need to display an initial reading quickly and the need to avoid successive readings showing a wide variation not consequent on any actual variation in the heartbeat rate, but on vagaries of the averaging process or lost and spurious pulse signals. A satisfactory result can be achieved by displaying readings based on successive groups of three to ten arterial pulses.

In a first alternative embodiment, suitable for use where blood pressure measurement is not required, the transducer 2 can be omitted, and the signal from the electrodes 7 used in place of the signal from the transducer 2 for deriving the heartbeat rate signal.

In a second alternative embodiment, suitable for use where only blood pressure measurement is required, only the signal representing the mean blood pressure is derived. This embodiment need not be housed in a watch casing, although it is preferably provided in a hand-held form.

## CLAIMS

1. A cardiovascular monitor comprising:

a casing;

a timing circuit (3) disposed in said casing for supplying a timing signal;

a transducer (2) associated with said casing for sensing arterial blood pulses at said transducer (2) and supplying an electric signal in dependence thereon;

electrode means (7) associated with said casing for sensing electrocardiographic R-waves at said electrode means (7) and supplying an electric signal in dependence thereon;

circuit means (4) for deriving from said electrode signal, said transducer signal and said timing signal an electric signal representing mean blood pressure; and

display means (6) mounted on said casing for displaying mean blood pressure in dependence on said blood pressure signal.

2. A monitor according to claim 1 wherein said casing is a watch casing and said monitor further comprises circuit means (5) for deriving from said transducer signal and said timing signal an electric signal representing heartbeat rate and display means (6) mounted on said watch casing for displaying the time of day in dependence on said timing signal, heartbeat rate in dependence on said heartbeat rate signal, and mean blood pressure in dependence on said blood pressure signal.

3. A monitor according to claim 1 or claim 2 wherein said transducer (3) comprises a light emitting diode and photosensor means to receive light originating from said light emitting diode and reflected from skin tissue.

4. A monitor according to claim 3 wherein said light emitting diode emits light of wavelength in the range 600 to 650 nm.

5. A monitor according to claim 3 wherein said photosensor means comprises a photodiode or a photodetector.

6.    A monitor according to claim 1 or claim 2 wherein said transducer (2) comprises a light source for transilluminating skin tissue and semiconductor detector means disposed to receive light from said light source reflected from skin tissue and capable of detecting variations in the level of said light reflected from said skin tissue so as to produce said transducer signal which is responsive to changes in arterial blood flow in said skin tissue, said detector means also being capable, on exposure to ambient light, of supplying a recharging current for a power source (1);
and wherein said monitor further comprises:
a rechargeable electric power source (1);
means to supply said current from said detector means to said power source to recharge said power source (1); and
means to energise said timing circuit (3), light source, circuit means and display means (6) from said power source (1).

7.    A monitor according to claim 6 wherein said means for supplying said current to recharge said power source (1) includes voltage step-up means.

8.    A monitor according to claim 1 or claim 2 wherein said display means (6) comprises light emitting diodes.

9.    A monitor according to claim 1 or claim 2 wherein said display means (6) comprises liquid crystal cells.

10.    A cardiovascular monitor comprising:
a watch casing;
a timing circuit (3) disposed in said watch casing for supplying timing signals;
electrode means (7) associated with said watch casing for sensing electrocardiographic R-waves at said electrode means (7) and supplying an electric signal in dependence thereon;
circuit means (5) for deriving from said electrode signal and a said timing signal an electric signal representing heartbeat rate; and
display means (6) mounted on said watch casing for displaying the time of day in dependence on a said timing signal, and heartbeat rate in dependence on said heartbeat rate signal.

0021800

1/1

FIG. 1

FIG. 2